(19) Europäisches Patentamt
European Patent Office
Office européen des brevets

(11) **EP 1 908 820 A1**

(12) **EUROPEAN PATENT APPLICATION**

(43) Date of publication:
**09.04.2008 Bulletin 2008/15**

(51) Int Cl.:
*C12N 5/06* (2006.01)     *C12N 5/08* (2006.01)
*A61L 27/38* (2006.01)     *A61L 27/56* (2006.01)
*C08L 67/04* (2006.01)

(21) Application number: **06076845.4**

(22) Date of filing: **06.10.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicants:
- **Stichting voor de Technische Wetenschappen**
  **3527 JP  Utrecht (NL)**
- **Vrije Universiteit Medisch Centrum (VUMC)**
  **1081 HV  Amsterdam (NL)**

(72) Inventors:
- **Breuls, Roel Gertrudis Martinus**
  **3573 AB Utrecht (NL)**
- **Helder, Martinus Nicolaas**
  **8226 RL Lelystad (NL)**

- **van Milligen, Florine Johanna**
  **1391 CA Abcoude (NL)**
- **Smit, Theodoor Henri**
  **5262 XB Vught (NL)**

(74) Representative: **van Loon, C.J.J. et al**
**c/o VEREENIGDE**
**Johan de Wittlaan 7**
**2517 JR  Den Haag (NL)**

Remarks:
The sequence listing, which is published as annex to the application documents, was filed after the date of filing. The applicant has declared that it does not include matter which goes beyond the content of the application as filed.

(54) **Stem cell and/or progenitor cell enrichment**

(57)     The invention provides a method for enriching a stem cell-containing cell population and/or a progenitor cell-containing cell population for stem cells and/or progenitor cells, the method comprising:
- providing said cell population with a scaffold comprising a copolymer comprising lactide and caprolactone,
- allowing cells to attach to said scaffold, and
- at least in part removing unattached cells.

EP 1 908 820 A1

Annex to the application documents - subsequently filed sequences listing

SEQUENCE LISTING

<110>  Technologiestichting STW

<120>  Stem cell and/or progenitor cell enrichment

<130>  P79022EP00

<140>  EP 06076845.4
<141>  2006-10-06

<160>  12

<170>  PatentIn version 3.3

<210>  1
<211>  20
<212>  DNA
<213>  Artificial Sequence

<220>
<223>  Primer used for real time PCR 18S forward

<400>  1
gtaacccgtt gaaccccatt                                                    20

<210>  2
<211>  21
<212>  DNA
<213>  Artificial Sequence

<220>
<223>  Primer used for real time PCR 18S reverse

<400>  2
ccatccaatc ggtagtagcc g                                                  21

<210>  3
<211>  18
<212>  DNA
<213>  Artificial Sequence

<220>
<223>  Primer used for real time PCR Runx-2 forward

<400>  3
atgcttcatt cgcctcac                                                      18

<210>  4
<211>  19
<212>  DNA
<213>  Artificial Sequence

<220>
<223>  Primer used for real time PCR Runx-2 reverse

<400>  4
actgcttgca gccttaaat                                                     19

```
<210>  5
<211>  22
<212>  DNA
<213>  Artificial Sequence

<220>
<223>  Primer used for real time PCR OPN forward

<400>  5
ttccaagtaa gtccaacgaa ag                                          22


<210>  6
<211>  22
<212>  DNA
<213>  Artificial Sequence

<220>
<223>  Primer used for real time PCR OPV reverse

<400>  6
gtgaccagtt catcagattc at                                          22


<210>  7
<211>  18
<212>  DNA
<213>  Artificial Sequence

<220>
<223>  Primer used for real time PCR Col 1 forward

<400>  7
aagccgaatt cctggtct                                               18


<210>  8
<211>  19
<212>  DNA
<213>  Artificial Sequence

<220>
<223>  Primer used for real time PCR Col 1 reverse

<400>  8
tccaacgaga tcgagatcc                                              19


<210>  9
<211>  20
<212>  DNA
<213>  Artificial Sequence

<220>
<223>  Primer used for real time PCR Col 2 forward

<400>  9
ggatgggcag aggtataatg                                             20
```

```
<210>  10
<211>  19
<212>  DNA
<213>  Artificial Sequence

<220>
<223>  Primer used for real time PCR Col 2 reverse

<400>  10
ggtcctttgg gtcctacaa                                              19


<210>  11
<211>  17
<212>  DNA
<213>  Artificial Sequence

<220>
<223>  Primer used for real time PCR Aggrecan forward

<400>  11
caactacccg gccatcc                                               17


<210>  12
<211>  21
<212>  DNA
<213>  Artificial Sequence

<220>
<223>  Primer used for real time PCR Aggrecan reverse

<400>  12
gatggctctg taatggaaca c                                          21
```

**Description**

The invention relates to the field of medicine.

Regenerative medicine, also referred to as reparative medicine or tissue engineering, often comprises regeneration and/or remodeling of tissue *in vivo* in order to repair, replace, maintain and/or enhance organ function, as well as engineering and growing functional tissue substitutes *in vitro,* preferably for implantation in vivo as biological substitutes for damaged and/or diseased tissues and/or organs. Regenerative medicine is a booming multidisciplinary field involving biology, medicine, and engineering. Regenerative medicine usually comprises (combinations of) three main building blocks: 1) biomaterials, 2) biologics (inductive stimuli) and 3) stem cells and/or progenitor cells. Biomaterials are designed to promote the organization, growth, and/or differentiation of cells in the process of forming functional tissue by providing structural support and/or biological containment. Biologics are commonly used to enhance cell proliferation and/or differentiation and for instance comprise growth factors, cytokines, hormones and/or mechanical signals. Another important element in regenerative medicine is the availability of regeneration-competent cells. The ability to harvest and/or procure high quantities of lineage specific cells or direct regeneration-competent progenitor cells towards the proper phenotype is desired for one step and multiple step interventions. Cells used in regenerative medicine are obtainable from various sources. Stem cells or progenitor cells (regeneration competent cells) are for instance recruited from tissue surrounding the implanted tissue construct, or are harvested from tissue that contains stem cells and/or progenitor cells such as bone marrow, skin, muscle, and adipose tissue. In general, the number of stem cells in any given tissue compartment is very low. For example, the stem cell incidence in bone marrow is estimated to be about 1 per $10^5$ cells, and other compartments harbour even lower stem cell numbers. Therefore, adipose tissue is preferably used. Adipose tissue has several advantages over bone marrow, such as (i) easy accessibility; (ii) minimal morbidity upon harvest; (iii) clinically relevant stem cell numbers are extracted from adipose tissue isolates; and (iv) higher stem cell proliferation rates than bone marrow mesenchymal stem cells (BM-MSCs). Adipose tissue derived mesenchymal stem cells (AT-MSCs) show multiple differentiation potential, i.e. they differentiate *in vitro* and *in vivo* towards adipogenic, osteogenic, chondrogenic, myogenic, ligamentous, and probably, neurogenic, endothelial, haematopoietic and cardiomyogenic phenotypes.

It is well established that the stromal cell fraction isolated from adipose tissue (SVF) is a heterogeneous mixture of cells including not only adipose tissue-derived mesenchymal stem cells (ASCs) but also a small number of hematopoietic cells, pericytes, endothelial cells and vascular smooth muscle cells. Stem cells and/or progenitor cells are often isolated by purification and culturing methods and subsequently attached to a scaffold. However, such purification and culturing methods require time consuming processing steps, while the (potency) properties of the stem cells and/or progenitor cells often diminish. From a clinical viewpoint, it would be desirable to seed the heterogenous mixture of stromal vascular fraction (SVF) cells onto a scaffold material directly after isolation, thereby avoiding lengthy purification and expansion procedures of the ASCs. This would make a cost efficient, so called one-step surgical procedure within reach wherein stem cells and/or progenitor cells are obtained from an individual, attached to a scaffold followed by transplantation of the scaffold into the individual. Since the ASCs are the cells intended to form new tissue, it is important that among all cells, at least the ASC fraction within the SVF adheres to the scaffold in sufficient quantities, preferably within a short time frame.

It is an object of the present invention to provide means and methods for enriching a cell population for stem cells and/or progenitor cells.

In a first aspect the invention provides a method for enriching a stem cell-containing cell population and/or a progenitor-containing cell population for stem cells and/or progenitor cells, the method comprising:

- providing said cell population with a scaffold comprising a copolymer comprising lactide and caprolactone,
- allowing cells to attach to said scaffold, and
- at least in part removing unattached cells.

According to the present invention, a scaffold comprising a copolymer comprising lactide and caprolactone is capable of enriching a cell population for stem cells and/or progenitor cells. It has surprisingly been demonstrated that, after incubation of said scaffold with a stem cell/progenitor cell-containing cell population, the percentage of stem cells and/or progenitor cells attached to said scaffold, relative to the total amount of cells, is higher than the percentage of stem cells and/or progenitor cells present in the original population. Hence, the resulting population of cells attached to said scaffold is enriched in stem cells and/or progenitor cells. The enriched population of cells is particularly suitable for use in regenerative medicine. Labor-intensive, risky, expensive and time-consuming *in vitro* expansion of stem cells and/or progenitor cells in the laboratory is no longer necessary.

A scaffold as used herein is a synthetic filler or carrier capable of supporting and/or carrying cells and/or tissue. Although not necessary, a scaffold preferably has an architecture for promoting formation, migration, residence and/or proliferation of cells and/or for providing a structural support during wound healing. For instance, for bone the pores of a scaffold preferably have a diameter of about 100-1000 $\mu$m, more preferably 200-600 $\mu$m, most preferably about

250-425 $\mu$m. For cartilage, the pores of a scaffold preferably have a diameter within the same range or lower, most preferably of about 75-300$\mu$ m. The interconnections preferably have a diameter of 50-200 $\mu$m, more preferably 120-150 $\mu$m. The ratio of surface to volume is preferably such that a porosity of at least 60%, preferably at least 70%, more preferably at least 80%, still more preferably at least 90% is obtained.

Stem cells and progenitor cells are undifferentiated cells capable of proliferation and capable of differentiation into another cell type. The distinction between stem cells and progenitor cells is rather theoretical and is related to the potency of the cells. Both stem cells and progenitor cells are capable of differentiating into another cell type, but stem cells are considered capable of differentiating into more different kinds of cell types as compared to progenitor cells. Stem cells as well as progenitor cells are CD34-positive cells. As used herein, the term "stem cell" also embraces "progenitor cell".

In a preferred embodiment cells from adipose tissue is enriched in stem cells and/or progenitor cells, because adipose tissue has several advantages over bone marrow, as described above. An advantage of the use of stem cells and/or progenitor cells from adipose tissue over the use of stem cells/progenitor cells from bone marrow is that, for obtaining adipose tissue, a very painful and uncomfortable bone marrow collection is not necessary. Most preferably a stromal vascular fraction of adipose tissue is enriched in stem cells and/or progenitor cells.

In one embodiment said adipose cells are obtained through resection and/or liposuction. This involves minimal discomfort, without long purification procedures and with high yield (300 cc aspiration generally produces a yield of 2 to 6 x $10^8$ cells). Aspiration of adipose tissue (liposuction) is carried out by use of any method known for that purpose. The aspirate can be obtained from different parts of the body such as from abdomen, thigh or buttocks. A skilled person will easily be able to determine that the amount and quality of the harvested stem cells can vary according to the site of collection and depth of the aspiration of the adipose tissue. Also, the minimum aspiration volume is easily determined to obtain a sufficient amount of stem cells.

In a preferred embodiment a scaffold comprising poly(D,L-lactide-co-caprolactone), poly(L,L-lactide-co-caprolactone) and/or a mixture thereof is used. Preferably said scaffold comprises poly-eta-caprolactone. The L-isomers as well as the D-isomers of lactic acid are suitable for preparing a scaffold for use in a method according to the invention. In a preferred embodiment a mixture of L-lactic acid and D-lactic acid is used in the preparation of a polylactide-co-caprolactone copolymer scaffold for use in a method according to the invention. A weight ratio of L-lactic acid : D-lactic acid in the scaffold is preferably in the range from about 20:80 to 80:20, more preferably 60:40 to 40:60. Most preferably, the ratio L-lactic acid : D-lactic acid in the scaffold is about 50:50. For an overview of the D and L isomers of lactic acids, see for instance page 134 of Wuisman and Smit, Eur Spine J. (2006) 15: 133-148. Poly(lactide-co-caprolactone) scaffolds are biodegradable and available in the art. Methods for generating and using such scaffolds are known, see for instance (Wuisman and Smit, Eur Spine J. (2006) 15: 133-148). The use of such scaffolds for enriching a cell population for stem cells and/or progenitor cells has not been described. Weight ratios of lactide : caprolactone in the scaffold are preferably in the range from about 50:50 to 90:10. More preferably, a weight ratio of lactide : caprolactone in the scaffold in the range from about 60:40 to 80:20 is used. Most preferably, the ratio lactide : caprolactone in the scaffold is about 70:30. A scaffold comprising poly(D,L-lactide-co-caprolactone), poly(L,L-lactide-co-caprolactone) or a mixture thereof, wherein the weight ratios of lactide : caprolactone are within the above mentioned ranges are particularly suitable for enriching a cell population for stem cells and/or progenitor cells. Further provided is thus a method according to the invention, wherein the ratio lactide : caprolactone in the scaffold is in the range from about 50:50 to 90:10, preferably from about 60:40 to 80:20, most preferably about 70:30.

With a method according to the present invention, a population is enriched for stem cells and/or progenitor cells within a short time frame. Surprisingly, a stem cell/progenitor cell-enriched cell population is attached to said scaffold within a time frame of between ten minutes and one hour. Within this short time frame a sufficient yield is obtained. This provides the advantage that a procedure has become possible wherein stem cells and/or progenitor cells of an individual are harvested (preferably by resection and/or liposuction of adipose tissue followed by incubation of the harvested cell population with a scaffold comprising a copolymer comprising lactide and caprolactone) and directly returned to a defect site within a single surgical procedure because it is not necessary to expand the stem cells and/or progenitor cells by means of laboratory culture. Hence, a one-step surgical procedure is possible. Not only is this concept cost effective but it is also beneficial for the patient, since a second surgical intervention is avoided.

Further provided is therefore a method according to the invention wherein the cells are allowed to attach to the scaffold during a time frame between ten minutes and one hour. Preferably, said cells are allowed to attach to said scaffold during a time frame between ten minutes and thirty minutes. Even within this short period a sufficient yield is obtained.

A stem cell/progenitor cell-containing cell population is incubated with a scaffold as used herein using any conventional method known in the art. A scaffold is preferably incubated with a culture medium comprising a cell population of interest. Suitable culture media for seeding cells are well known in the art. For instance, cells are seeded in medium comprising DMEM, optionally together with FBS and/or L-glutamine.

Once stem cells and/or progenitor cells are attached to a scaffold, proliferation of these cells is preferably induced and/or enhanced in order to obtain a tissue of interest. This is for instance performed using at least one growth factor such as a TGF-β and/or a BMP, a polyamine and/or a steroid such as vitamin D and/or dexamethason. Further provided is therefore a method according to the invention, further comprising inducing and/or enhancing proliferation of stem cells and/or progenitor cells attached to said scaffold. In one preferred embodiment chondrogenic and/or osteogenic differentiation of stem cells and/or progenitor cells is induced and/or enhanced.

A scaffold comprising attached stem cells and/or progenitor cells obtainable by a method according to the invention is also herewith provided, as well as a use of a scaffold comprising a copolymer comprising lactide and caprolactone for enriching a stem cell-containing cell population and/or a progenitor cell-containing cell population for stem cells and/or progenitor cells. Said scaffold preferably comprises a porous matrix comprising a copolymer, said copolymer comprising at least one lactide and caprolactone, and stem cells and/or progenitor cells. As described above, said stem cells and/or progenitor cells are preferably from adipose tissue, amongst other things in view of the relative high amounts of stem cells and/or progenitor cells present in adipose tissue and the relatively little discomfort involved in obtaining adipose tissue from a patient. In the context of the present invention, the term "porous" is defined as having cavities (pores) of a sufficient dimension to accommodate a cell and to let a cell suspension penetrate through the cavities into the material. The cavities may for instance be the interstitial volume between fibers or particles of the scaffold or the cage or the holes in a substantially homogeneous, open-solid structure.

DETAILED DESCRIPTION

A method according to the invention is suitable for use in a wide variety of regenerative medicine applications. Preferred applications are in the field of cartilage and/or bone implant. One aspect of the present invention relates to this preferred embodiment, which is further outlined below.

A scaffold according to the invention, comprising a cell population which is enriched in stem cells and/or progenitor cells, is suitable for use in cartilage and/or bone repair. In this embodiment, differentiation of the stem cells and/or progenitor cells attached to the scaffold into cartilage and/or bone is preferably induced and/or enhanced. Preferably, the stem cells/progenitor cells are induced to differentiate to osteoblasts and/or chondrocytes by use of cartilage growth factors and/or bone growth factors (cartilage growth factors and/or bone growth factors also being called cartilage and/or bone growth factors).

To this end, cartilage and/or bone growth factors may *inter alia* be added to the scaffold in order to induce the differentiation of the stem cells and/or progenitor cells to osteoblasts and/or chondrocytes therein. Also, the stem cells and/or progenitor cells can first be brought into contact with cartilage and/or bone growth factors, and then be added to the scaffold. In alternative embodiments, the induction of the differentiation of the stem cells and/or progenitor cells is carried out on the scaffold by means of cartilage and/or bone growth factors prior to surgical implantation or even after the implant has been introduced into the patient.

Cartilage and/or bone growth factors are specific proteins capable of influencing synthesis and degradation processes in the body by regulating cell growth and cell function. The cartilage and/or bone-inducing (new cartilage and/or bone development in a cartilage and/or bone-free environment) growth factors, including the bone morphogenetic proteins (BMPs) can make an undifferentiated stem cell differentiate to a progenitor bone cell (preosteoblast). Other factors affecting osteogenic and/or chondrogenic differentiation include transforming growth factor β (TGF-B), insulin-like growth factor (IGF), and platelet-derived growth factor (PDGF). They are capable of making the number of differentiated bone and/or cartilage cells increase in number (proliferate) or differentiate further to a bone matrix-forming cell (osteoblast) or cartilage-forming cell (chondrocyte).

In principle, both types of growth factors can be used in embodiments according to the present invention. Many growth factors as described in various overview publications (Bonewald, 2002, In Bilezikian et al., (eds) Principles of Bone Biology, Academic Press, San Diego, pp.903-18; Rosen and Wozney, 2002, In Bilezikian et al., (eds) Principles of Bone Biology, Academic Press, San Diego, pp. 919-28; Reddi, 2000, Tissue Eng. 6:351-9; Boden, 2000, Tissue Eng. 6:383-99; Deuel et al., 2002 Arch. Biochem. Biophys. 397:162-172) can be used in embodiments according to the present invention. Preferably, growth factors from the group of TGF-βs (*inter alia* TGF-β1-5) and BMPs are used. With great preference, one or more of the growth factors of TGF-B, osteoblast stimulating factor-1 (Osf-1), recombinant human BMP-2 (rhBMP-2) and LIM mineralization protein-1 (LMP-1) are used.

Growth factors are obtained in at least two manners. One method is the extraction from tissues or tissue fluids (autologous, allologous or xenologous), the other is synthesizing by means of gene transcription of modified DNA in dividing cells in culture (recombinant techniques). An example of the first method is PRP (platelet-rich plasma), in which the body's own growth factors (mainly PDGF and TGF-β) are obtained from blood.

For preparing an artificial cartilage and/or bone implant according to the invention, a resorbable porous scaffold is very suitably combined with an osteo-inductive and/or chondro-inductive composition as described hereinabove to provide an artificial bone implant according to the invention. Thus, a scaffold according to the invention is very suitably combined with an osteo-inductive and/or chondro-inductive composition, for instance by means of diffusion, electrophoresis, centrifugal forces or cross-linking.

An artificial cartilage and/or bone implant according to the invention may optionally be processed further after manufacturing thereof, for instance by grinding, polishing, providing attachment possibilities such as screw holes or hooks, or crushing.

An artificial cartilage and/or bone implant according to the invention may have a complex geometrical shape. The shape may have, for instance, cuts, internal cavities, recesses or protrusions. The cartilage and/or bone implant may be shaped such that a specific bone or specific bones or parts thereof or spaces between bones or cavities in bones can be replaced or filled therewith. The cartilage and/or bone implant can be dimensioned and shaped prior to surgery specially for a particular patient. Also, the cartilage and/or bone implant may have a simple shape, such as a block, which is intended to be shaped by a surgeon during a surgical operation. The cartilage and/or bone implant can be made to fit for a cartilage and/or bone defect and may be tapered or finished with oblique sides or be provided with engaging parts for enhancing the fit.

A cartilage and/or bone-surgical method according to the invention comprises any method for repairing a cartilage and/or bone defect in a vertebrate.

Further provided is thus a scaffold according to the invention, which is an artificial cartilage implant and/or bone implant. Said artificial cartilage implant and/or bone implant preferably further comprises at least one growth factor such as a TGF-β and/or a BMP, a polyamine and/or a steroid such as vitamin D and/or dexamethason.

As described above, an artificial cartilage implant and/or bone implant according to the invenion preferably comprises a scaffold comprising poly(D,L-lactide-co-caprolactone), poly(L,L-lactide-co-caprolactone) and/or a mixture thereof, since these scaffolds are particularly suitable for enriching a cell population for stem cells and/or progenitor cells. The ratio lactide : caprolactone in the scaffold is preferably in the range from about 50:50 to 90:10, more preferably in the range from about 60:40 to 80:20, most preferably about 70:30. An artificial cartilage implant and/or bone implant is preferably produced with a method according to the invention, as described before. An artificial cartilage implant and/or bone implant obtainable by a method according to the invention is therefore provided.

An artificial cartilage implant and/or bone implant according to the invention is particularly suitable for use as a medicament, especially for treatment of cartilage damage and/or bone damage.

Further provided are therefore an artificial cartilage implant and/or bone implant according to the invention for use as a medicament, as well as a use of an artificial cartilage implant and/or bone implant according to the invention for the preparation of a medicament for treatment of cartilage damage and/or bone damage.

A use of an artificial cartilage implant and/or bone implant according to the invention for cartilage-surgical operations and/or bone-surgical operations, in particular spinal fusions, is also provided as well as a use of an artificial cartilage implant and/or bone implant according to the invention for inducing the growth of new cartilage tissue and/or bone tissue.

Further provided is a method for carrying out cartilage surgery and/or bone surgery, comprising implanting an artificial cartilage implant and/or bone implant according to the invention into cartilage and/or a bone of a patient.

One aspect of the invention furthermore provides a method for inducing new cartilage tissue and/or bone tissue in a vertebrate in need thereof, the method comprising:

- removing adipose tissue from a vertebrate,
- providing cells from said adipose tissue to a scaffold comprising a copolymer comprising at least one lactide and caprolactone,
- allowing cells to attach to said scaffold,
- at least in part removing unattached cells, and
- introducing said scaffold into said vertebrate in need of new cartilage tissue and/or bone tissue. Said scaffold preferably comprises poly(D,L-lactide-co-caprolactone), poly(L,L-lactide-co-caprolactone) and/or a mixture thereof. The ratio lactide : caprolactone in said scaffold is preferably in the range from about 50:50 to 90:10, more preferably from about 60:40 to 80:20, most preferably about 70:30.

The invention is further explained in the following examples. These examples do not limit the scope of the invention, but merely serve to clarify the invention.

Examples

Materials and Methods

Tissue sampling

Human subcutaneous adipose tissue samples were obtained as waste material after elective surgery and donated upon informed consent of the patients by the department of Plastic Surgery from the VU University Medical Center. Adipose tissue was harvested from the abdomen or hip and thigh region of healthy females, using either resection or tumescent liposuction.

**Isolation of the SVF of adipose tissue.**

Adipose tissue was weighed and extensively washed with phosphate-buffered saline (PBS). Resected material was first cut into small pieces, using a surgical scalpel. Tissue was enzymatically digested with 0.1% Collagenase A (Roche Diagnostics GmbH, Mannheim, Germany) in PBS-1% BSA, for 30 min at 37°C with intermittent shaking. Enzyme activity was then neutralized by adding Dulbecco's Modified Eagle's Medium containing 4.5g/l glucose (DMEM; BioWhittaker, Cambrex, Verviers, Belgium) and supplemented with 10% FBS. The digested adipose tissue was centrifuged for 10 min at 600 g. The cell containing pellet was resuspended in PBS and passed through a 200 $\mu$m nylon mesh to remove debris. Subsequently, cells were subjected to a ficoll density centrifugation step ($\rho$ = 1,077 g/ml, Osmolarity 290 $\pm$ 15 mOsm; AXIS-SHIELD Poc as, Oslo, Norway) to remove contaminating erythrocytes. The cell containing interface was harvested and washed with DMEM-10% FBS. Viability of the cells was assessed using trypan blue exclusion assay. $5 \times 10^6$ cells were resuspended in a mixture (1:1) of DMEM-10% FBS and Cyroprotective medium (Freezing Medium, BioWhittaker, Cambrex, Verviers, Belgium), frozen under "controlled rate" conditions in a Kryosave (HCI Cryogenics BV., Hedel, The Netherlands), and stored in the vapor phase of liquid nitrogen according to standard practice in the Department of Pathology of the VU University Medical Center, and following the guidelines of current Good Manufacturing Practice.

**70:30 Poly(D,L-lactide-co-caprolactone) Scaffold**

70:30 Poly(D,L-lactide-co-caprolactone) scaffolds, which are approved by the FDA for cartilage and bone repair (Ishaugh-Riley *et al.* 1999), were kindly provided by KA Thomas (Cytori Therapeutics, San Diego, Cal). The scaffold evokes only a minor foreign body reaction and is degraded by water, releasing non-toxic waste products. The scaffold has pore sizes between 250-425 $\mu$m and a porosity of 85-90%. In these studies scaffolds of 6 mm diameter and 1 mm height were used. Before use, scaffolds were rinsed with 70% ethanol, washed with PBS and incubated overnight in DMEM supplemented with 10% FBS, 100 U/ml penicillin, 100 $\mu$g/ml streptomycin and 2 mM L-glutamine (all from Gibco, Invitrogen, California, USA) in a humidified incubator (37°C and 5% $CO_2$).

**Kinetics of cell adhesion to 70:30 Poly(D,L-lactide-co-caprolactone) scaffold and plastic.**

For the assessment of the kinetics of cell adhesion, 50.000 SVF cells were seeded per scaffold in 30 $\mu$l of control medium (DMEM supplemented with 10% FBS, 100 U/ml penicillin, 100 $\mu$g/ml streptomycin and 2 mM L-glutamine). The cells were allowed to attach to the scaffolds undisturbed in a humidified incubator (37°C and 5% $CO_2$) for 5', 10', 20', 30', 1 hr, 2 hrs, 4 hrs, 8 hrs and 24 hrs, respectively. To the cells that were allowed to attach for more than 1 hour, 750 $\mu$l of control medium was added after 1 hour. In parallel, 50.000 SVF cells were seeded per well of a 24-wells plate in 750 $\mu$l of control medium. The cells were allowed to attach to the plastic culture wells for 1, 2, 4, 8 and 24 hrs, respectively. At each time point, scaffolds or culture wells were rinsed with PBS to removed unattached cells and the number of attached cells was quantified by assaying for DNA, using a CyQUANT Cell Proliferation Assay Kit (Invitrogen).

**Flow cytometry**

Single cell suspensions of cryopreserved, freshly isolated adipose tissue-derived SVF cells and cultured ASCs (passage 4) were phenotypically characterized using FACS (FACSCalibur, Becton Dickinson, USA). Cells were washed in FACS buffer (PBS containing 1% BSA and 0,05% sodium azide) and per sample $1 \times 10^5$ cells were incubated on ice with the optimal dilution of conjugated mAb in FACS buffer. All mAbs were of the IgGl isotype. Cell populations were identified in two or three colour stainings, using allophycocyanin-conjugated mAbs against CD34 (clone 8612; BD Biosciences, San José, CA), FITC-conjugated mAbs against platelet endothelial cell adhesion molecule (PECAM or CD31, BD), and PE-conjugated mAbs against melanoma cell adhesion molecule (MEL-CAM or CD146, mAb 16985H, Chemicon Temecula, CA) and CD45 (BD Biosciences, San José, CA). After a 30-min incubation, cells were washed twice with FACS buffer. Non-specific fluorescence was determined by incubating cells with conjugated mAb antihuman IgG1 (DAKO Cytomation, Glostrup, Denmark). Flow cytometry data were analysed using CELLQUEST software (Becton Dickinson).

**Phenotypical characterization of SVF cells adhering to scaffold.**

Per scaffold 200 000 SVF cells were seeded and after 1 hr cells were washed with PBS and fixed in 4% paraformaldehyde for 30 min. The scaffolds were then washed two times with PBS and once with PBS-0.1% BSA and incubated for 1 hour with a 1:50 dilution of mAbs against CD34, CD31, CD45 (all from BD Biosciences, San José, CA) and CD146 (Chemicon Temecula, CA), respectively, in PBS-0.1% BSA. The scaffolds were then washed 3 times with PBS-0.1% BSA and incubated with a 1:500 dilution of biotin-conjugated rabbit antibodies against mouse immunoglobulins RaMBioF(ab')$_2$ (DAKO Cytomation, Glostrup, Denmark) in PBS-0.1% BSA for 1 hour. After 3 washings with PBS, a 1: 500 dilution of Streptavidin Alexa Fluor™ 633 conjugate (Molecular Probes. Eugene, Oregon, USA) was added for 1 hour. The scaffolds were washed 3 times with PBS and nuclei were stained with 7.5 $\mu$M propidium iodide during 15 min. After washing 3 times with PBS, antibody staining of the cells was analyzed by confocal microscopy, using a Leica TCS SP5 (Leica Microsystems, Wetzlar, Germany). Topview projections of the scaffold were made by taking a z-stack (50 images) of the scaffold, consisting of horizontal cross sections that were subsequently stacked on top of each other, using the maximum projection algorithm of the Leica confocal software. The percentage of cells that adhered to the scaffolds after 1 hour incubation and stained positive for CD34, CD31, CD146 and CD45, respectively, were quantified by previously developed image analysis software [Breuls et al, 2003]. Randomly chosen volumes of 1.5x1.5x1mm were counted. All experiments were performed at least in triplicate using cells of three different donors.

**Proliferation of SVF cells in the scaffold**

Per scaffold 50 000 SVF cells were seeded in 30 $\mu$l of control medium. After 1 hr of adherence, unattached cells were washed away and scaffolds were incubated during 2, 6, 10 and 14 days, respectively, in control medium in a humidified incubator (37°C and 5% CO$_2$). At each time point, scaffolds were rinsed with PBS and the number of cells in the scaffold was quantified by assaying for DNA, using a CyQUANT Cell Proliferation Assay Kit (Invitrogen). To determine the population doubling time cell numbers were plotted against the number of days cultured, and the logarithmic growing phase of the cells was determined. The population doubling time was calculated using the formula:

$$Population\ Doubling\ Time = \frac{Days\ in\ logarithmic\ phase}{\frac{log(N2) - log(N1)}{log\ 2}}$$

where $N1$ is the number of cells at the beginning of the logarithmic growing phase, and $N2$ the number of cells at the end of the logarithmic growing phase.

**Chondrogenic and osteogenic differentiation**

For chondrogenic differentiation per scaffold 500 000 SVF cells were seeded in 30 $\mu$l of chondrogenic medium, consisting of DMEM, containing ITS+™ Premix (final concentration in medium when diluted 1:100 Insulin, 6.25 $\mu$g/ml, Transferrin, 6.25 $\mu$g/ml, Selenous Acid, 6.25 ng/ml, Bovine Serum Albumin, 1.25 mg/ml, Linoleic Acid, 5.35 $\mu$g/ml; BD, USA), 20 ng/ml transforming growth factor-$\beta_1$ (TGF-$\beta_1$. Biovision, ITK-diagnostics), 100 nM dexamethasone only on the 1$^{st}$ day, 1% FCS, 25$\mu$M ascorbate-2-phosphate (Sigma), 100 U/ml penicillin, 100$\mu$g/ml streptomycin and 2 mM L-glutamine. For osteogenic differentiation per scaffold 500 000 SVF cells were seeded in 30 $\mu$l of osteogenic medium, consisting of control medium supplemented with 10 mM 6-glycerol phosphate, 50 $\mu$g/ml ascorbic acid, and 100 ng/ml bone morphogenetic protein 2 (BMP-2. Peprotech EC LTD, London, UK). After 1 hour 750 $\mu$l of additional medium was added. Chondrogenic media were changed every other day, osteogenic media twice a week.

**Reverse transcription polymerase chain reaction**

Total RNA from the cells was isolated using TRIzol® reagent (Invitrogen, Carlsbad, CA, USA). cDNA synthesis (GeneAmp® PCR System9700, PE Applied Biosystems, CA, USA) was performed using 0.5-1 $\mu$g total RNA in a 20 $\mu$l reaction mix containing 5 Units of Transcriptor Reverse Transcriptase (Roche Diagnostics), 0.08 A$_{260}$ units random primers (Roche Diagnostics) and 1 mM of each dNTP (Invitrogen). The cDNA was stored at -80 °C prior to real-time PCR.
Real-time PCR reactions were performed using the SYBRGreen reaction kit according to the manufacturer's instructions (Roche Diagnostics) in a LightCycler (Roche Diagnostics). Primers (Invitrogen) used for real-time PCR are listed in Table 1. For real-time PCR, the values of relative target gene expression were normalized for relative 18S housekeeping gene expression.

Table 1: Primers used for real time PCR

| Target gene | Primer sets | Expected product size, bp |
|---|---|---|
| 18S | forward 5'-gtaacccgttgaaccccatt-3'<br>reverse 5'-ccatccaatcggtagtagccg-3' | 151 |
| Runx-2 | forward 5'-atgcttcattcgcctcac-3'<br>reverse 5'-actgcttgcagccttaaat-3' | 156 |
| OPN | forward 5'-ttccaagtaagtccaacgaaag-3'<br>reverse 5'-gtgaccagttcatcagattcat-3' | 181 |
| Col 1$\alpha$l | Forward -5'-aagccgaattcctggtct-3'<br>Reverse -5'-tccaacgagatcgagatcc-3' | 195 |
| Col2A1VARA | forward 5'-ggatgggcagaggtataatg-3'<br>reverse 5'ggtcctttgggtcctacaa-3' | 255 |
| Aggrecan | forward 5'caactacccggccatcc-3'<br>reverse 5'-gatggctctgtaatggaacac-3' | 160 |

## Results

### Kinetics of SVF cell adhesion

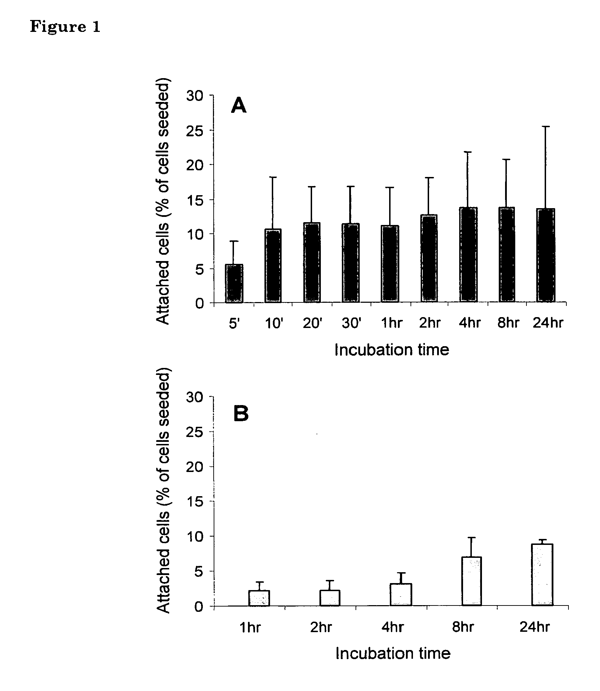

To investigate in what time frame the freshly isolated SVF cells adhered to the scaffold, the percentage SVF cells adhering to the PDLL-ε-caprolactone scaffold was monitored within time. As a reference we compared this with the adherence of SVF cells to conventional plastic polystyrene culture wells. SVF cells already attached to the scaffold within 5-10 min and adherence increased to about 10% after 10 min (figure 1). The maximum number of adhered cells was 14% $\pm$ 6.9% (mean $\pm$ SD) and occurred after 8h. The large standard deviations in figure 1A are due to a single donor for which a maximum of only 8% percent of the cells adhered. For the other 2 donors maximum binding was 22% and 27%, respectively.

In plastic culture wells (figure 1B) only few cells were detected after 1hr (2% $\pm$ 1.3%) and 2 hr (2% $\pm$ 1.4%) of adherence and a maximum binding of 9% $\pm$ 0.71% was reached between 8 and 24 hrs.

### Phenotypical characterization of SVF cells adhering to the scaffold.

In a previous study, the frequencies of ASC-like cells and other cell populations within the SVF of adipose tissue were determined using flow cytometry. These results are summarized in Table 2.

Table 2

| Cell type | Phenotype | Frequency in SVF (%) mean $\pm$ SD (n=5) |
|---|---|---|
| ASC-like | CD34$^+$CD31$^-$CD45$^-$ | 34.6 $\pm$ 17.8 |
| Endothelial cell | CD34$^+$CD31$^+$CD146$^+$ | 12.2 $\pm$ 9.5 |
| Hematopoietic stem cell-like | CD34$^+$CD45$^+$ | 5.2% $\pm$ 1.3% |
| Vascular smooth muscle cell/ pericyte | CD34$^-$CD31$^-$CD146$^+$ | 10.3 $\pm$ 9.9 |
| Leucocyte | CD45$^+$CD34$^-$ | 14.2 $\pm$ 2.3 |
| Unidentified | | 23.5 $\pm$ 10.1 |

When SVF cells are seeded onto the scaffold all these cell populations may potentially adhere. Therefore, we investigated which cell populations in the SVF did adhere to the scaffold using 3D confocal microscopy by automated counting of fluorescently labeled cells for the respective CD markers.

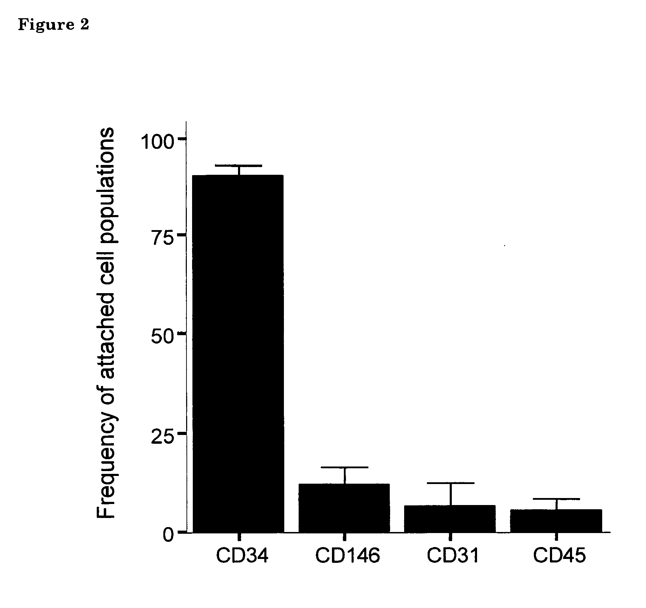

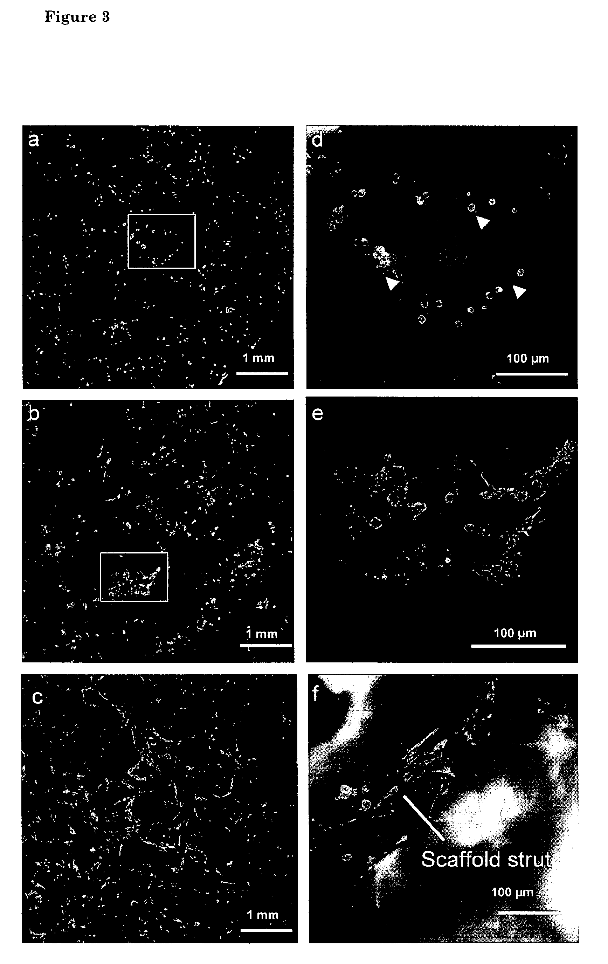

Surprisingly, 1 hour after seeding of the SVF, 89.9% $\pm$ 2.4% (mean $\pm$ SD, n=3) of the cells binding to the scaffold was CD34-positive (figure 2, figure 3a). These CD34-positive cells may represent the ASCs (CD34$^+$CD31$^-$CD45$^-$) endothelial cells (CD34$^+$CD31$^+$) and hematopoietic stem cells (CD34$^+$CD45$^+$). However, only low frequencies of CD31-positive (6.5% $\pm$ 5.8%) and CD45-positive (5.6% $\pm$ 2.9%) cells were detected in the scaffold, demonstrating that only few endothelial cells and hematopoietic stem cells bind to the scaffold. It also demonstrates that at least 89.9%-6.5%-5.6% = 77.8% of the attached cells has an ASC-like phenotype (CD34$^+$CD31$^-$CD45$^-$).

Also the frequency of CD146-positive cells in the scaffold was low (12.0% $\pm$ 4.4%). Since CD146 is expressed on endothelial cells, vascular smooth muscle cells and pericytes this demonstrates that in addition to endothelial cells (6,5% CD31$^+$) some vascular smooth muscle cells (CD34$^-$CD146$^+$) bind to the scaffold.

**Distribution, morphology and phenotype of ASCs in the scaffold**

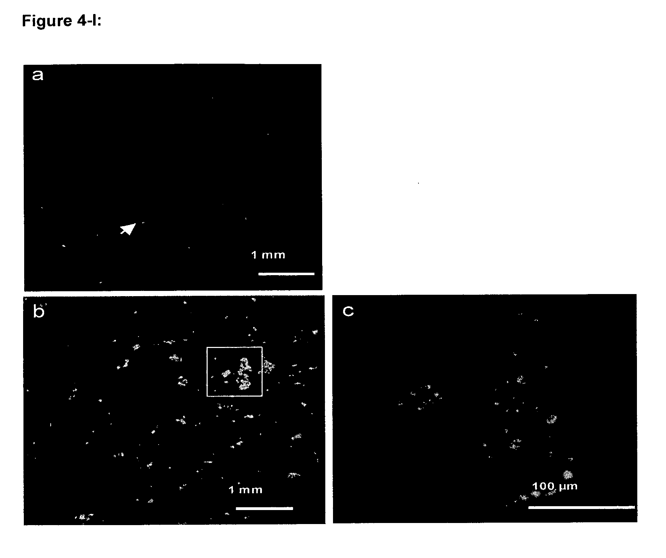

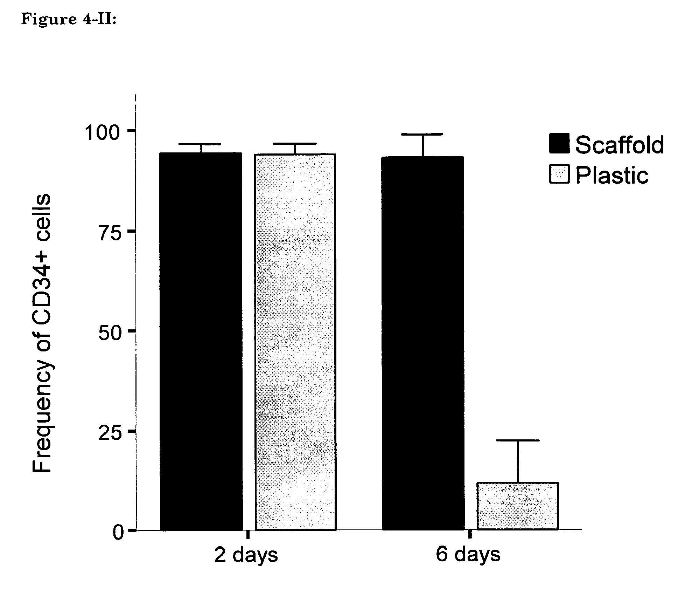

The distribution, morphology and surface marker expression of the adhered SVF cells are shown in figure 3 and figure 4. In figure 3, the CD34-positive cells are shown, 1hour, 2 days and 6 days after seeding of the SVF on the scaffold. After 1 hour, the CD34-positive cells still had a round morphology and adhered to the interior pores of the scaffold (figure 3a,d). Only a small percentage of cells was CD34-negative (see arrow-heads in figure 3d). Interestingly, after two days occasionally networks of interconnected cells were seen that attached to the scaffold material (figure 3b, e). After 6 days, the cells adopted a more fibroblast-like morphology (figure 3c) and covered the pores (figure 3c) and struts (figure 3f) of the scaffold. Cells were homogeneously distributed and a clear increase in cell numbers was seen after 6 days.

One hour after seeding, only few of the attached cells were stained positively for CD31 (Figure 4-I a). However, after 6 days, occasionally clusters of CD31-positive cells were seen (figure 4-I b,c). After 1 hour also few CD146 and CD45 positive cells were detected (see also figure 2) in the scaffold. After 2 and 6 days these cell populations were not detected any more (images not shown).

Interestingly, when monitoring the expression of CD34 of the adhered SVF cells in time we found that the expression of CD34 remained high over 6 days (figure 3c). When compared to ASCs cultured in a plastic culture well, this is remarkable as we (Figure 4-II) and others found that the expression of CD34 is rapidly lost upon *in vitro* culture in plastic culture wells:

**Proliferation of SVF cells in the scaffold**

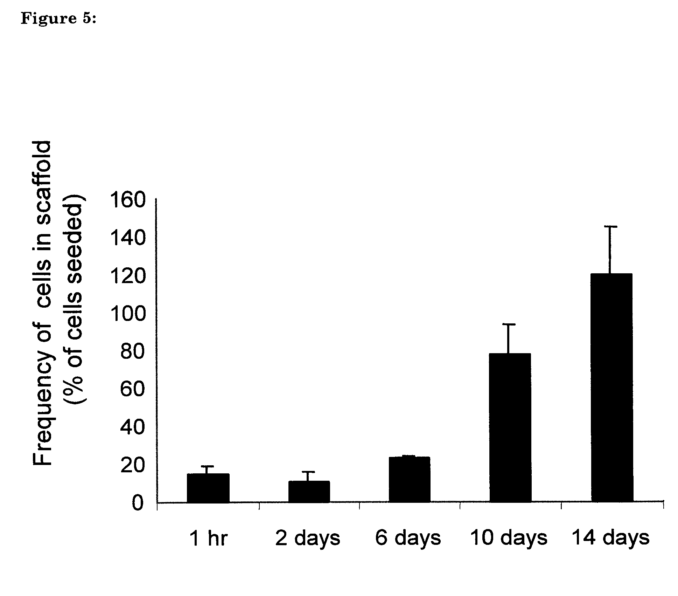

To answer the question whether the freshly isolated SVF cells did proliferate in the scaffold, we monitored the number of cells in the scaffold in time (figure 5). One hour after seeding of 50,000 SVF cells, 15% $\pm$ 4.2% (mean $\pm$ SD) of the SVF cells (7,500 cells) had adhered to the scaffold. This is comparable to the frequency of SVF cells that initially adhered after 1 hour attachment time point in the kinetics studies (figure 1). The number of cells increased from day 2 to 14, resulting in a more than 8-fold increase in the number of cells in the scaffold (60,250 cells), demonstrating that proliferation occurred in the scaffold. The mean population doubling time of the cells in the logarithmic growing phase was determined between day 6 and 10, and was 2.4 $\pm$ 0.3 days (mean $\pm$ SD).

**Osteogenic and chondrogenic differentiation of SVF cells in the scaffold.**

When SVF cells on the scaffold were cultured in control medium, cells were more or less homogeneously distributed throughout the scaffold. When cells were cultured in osteogenic medium cells were growing in close proximity of each other forming a monolayer (sheet) of cells. Strikingly, when cells were cultured in chondrogenic medium condensations of cells were formed resulting in large aggregates of cells and empty spaces in the scaffold. After 14 days of culture: RT-PCR and IHC.

Brief description of the drawings

Figure 1: Frequency of freshly isolated SVF cells which adhered to 70:30 Poly(D,L-lactide-co-caprolactone) scaffolds (A) and plastic culture wells (B) as monitored in time. Results are expressed as percentages from the total number of cells seeded onto the scaffolds (50.000) and plastic culture well

Figure 2: Frequency of adhered SVF cells staining positive for different CD markers, 1 hr after seeding onto a 70: 30 Poly(D,L-lactide-co-caprolactone) scaffold. Results are expressed as percentages from the total amount of adhered cells as counted from the number of PI stained nuclei.

Figure 3: Confocal images showing the distribution and morphology of CD34+ cells that adhered to the 70:30 Poly (D,L-lactide-co-caprolactone) scaffolds, 1 hour, 2 and 6 days after seeding of the SVF. Images are topview projections of a z-stack (50 images), which were taken as horizontal cross sections within the scaffold and subsequently stacked on top of each other. The green cells indicate positive staining for CD34. The red dots are the nuclei of all cells (either CD34$^+$ or CD34$^-$), which were stained with propidium iodide after fixation. Images (a-c) show an overview of the cell morphology and distribution within the scaffold at 1, 2, and 6 days at low magnification (10x objective lens). (d,e) show digital zoom views of representative areas in figure 3a,b. In figure 3d and 3f also the scaffold material is shown as a transmitted light image. Notice that after 6 days, the CD34 marker is still clearly visible on all cells (c,f). All experiments performed at least in triplicate, representative images shown.

Figure 4-I: Confocal images showing the distribution and morphology of CD31+ cells that adhered to the 70:30 Poly (D,L-lactide-co-caprolactone) scaffolds, 1 hour and 6 days after seeding of the SVF. The green cells indicate CD31-positive staining. (a) A very small amount of the CD31 cells had attached to the scaffold. After 6 days, occasionally a large cluster of CD31-positive cells was seen (b). These cells showed a round morphology (c).

Figure 4-II: Frequency of CD34$^+$ SVF cells, adhered to a 70:30 Poly(D,L-lactide-co-caprolactone) scaffold and plastic culture well, respectively, 2 days and 6 days after seeding. Results are expressed as percentages from the total number of adhered cells.

Figure 5: Frequency of freshly isolated SVF cells in a 70:30 Poly(D,L-lactide-co-caprolactone) scaffold as monitored in time, expressed as percentages from the total number of cells seeded onto the scaffolds (50,000 SVF cells).

References

1. Caplan, A.I. Mesenchymal Stem-Cells. Journal of Orthopaedic Research 9, 641, 1991.

2. Beresford, J.N., Bennett, J. H., Devlin, C., Leboy, P. S., and Owen, M. E. Evidence for An Inverse Relationship Between the Differentiation of Adipocytic and Osteogenic Cells in Rat Marrow Stromal Cell-Cultures. Journal of Cell Science 102, 341, 1992.

3. Berry, L., Grant, M. E., Mcclure, J., and Rooney, P. Bone-Marrow-Derived Chondrogenesis Invitro. Journal of Cell Science 101, 333, 1992.

4. Pittenger, M.F., Mackay, A. M., Beck, S. C., Jaiswal, R. K., Douglas, R., Mosca, J. D., Moorman, M. A., Simonetti, D. W., Craig, S., and Marshak, D. R. Multilineage potential of adult human mesenchymal stem cells. Science 284, 143, 1999.

5. Halvorsen, Y.D.C., Bond, A., Sen, A., Franklin, D. M., Lea-Currie, Y. R., Sujkowski, D., Ellis, P. N., Wilkison, W. O., and Gimble, J. M. Thiazolidinediones and glucocorticoids synergistically induce differentiation of human adipose tissue stromal cells: Biochemical, cellular, and molecular analysis. Metabolism-Clinical and Experimental 50, 407, 2001.

6. Mizuno, H., Zuk, P. A., Zhu, M., Lorenz, H. P., Benhaim, P., and Hedrick, M. H. Myogenic differentiation by human processed lipoaspirate cells. Plastic and Reconstructive Surgery 109, 199, 2002.

7. Zuk, P.A., Zhu, M., Mizuno, H., Huang, J., Futrell, J. W., Katz, A. J., Benhaim, P., Lorenz, H. P., and Hedrick, M. H. Multilineage cells from human adipose tissue: Implications for cell-based therapies. Tissue Engineering 7, 211, 2001.

8. Zuk, P.A., Zhu, M., Ashjian, P., De Ugarte, D. A., Huang, J. I., Mizuno, H., Alfonso, Z. C., Fraser, J. K., Benhaim, P., and Hedrick, M. H. Human adipose tissue is a source of multipotent stem cells. Molecular Biology of the Cell 13, 4279, 2002.

9. Wickham, M.Q., Erickson, G. R., Gimble, J. M., Vail, T. P., and Guilak, F. Multipotent stromal cells derived from the infrapatellar fat pad of the knee. Clinical Orthopaedics and Related Research 196, 2003.

10. Ashjian, P.H., Elbarbary, A. S., Edmonds, B., DeUgarte, D., Zhu, M., Zuk, P. A., Lorenz, H. P., Benhaim, P., and Hedrick, M. H. In vitro differentiation of human processed lipoaspirate cells into early neural progenitors. Plastic and Reconstructive Surgery 111, 1922, 2003.

11. Safford, K.M., Hicok, K. C., Safford, S. D., Halvorsen, Y. D. C., Wilkison, W. O., Gimble, J. M., and Rice, H. E. Neurogenic differentiation of murine and human adipose-derived stromal cells. Biochemical and Biophysical Research Communications 294, 371, 2002.

12. Rangappa, S., Fen, C., Lee, E. H., Bongso, A., and Wei, E. S. K. Transformation of adult mesenchymal stem cells isolated from the fatty tissue into cardiomyocytes. Annals of Thoracic Surgery 75, 775, 2003.

13. Seo, M.J., Suh, S. Y., Bae, Y. C., and Jung, J. S. Differentiation of human adipose stromal cells into hepatic lineage in vitro and in vivo. Biochemical and Biophysical Research Communications 328, 258, 2005.

14. Cowan, C.M., Shi, Y. Y., Aalami, O. O., Chou, Y. F., Mari, C., Thomas, R., Quarto, N., Contag, C. H., Wu, B., and Longaker, M. T. Adipose-derived adult stromal cells heal critical-size mouse calvarial defects. Nature Biotechnology 22, 560, 2004.

15. Helder, M.N., Knippenberg, M., Klein-Nulend, J., and Wuisman, P. I. J. M. Stem cells from adipose tissue allow challenging new concepts for regenerative medicine. Tissue Engineering accepted, 2006.

16. Ishaug-Riley, S.L., Okun, L. E., Prado, G., Applegate, M. A., and Ratcliffe, A. Human articular chondrocyte adhesion and proliferation on synthetic biodegradable polymer films. Biomaterials 20, 2245, 1999.

17. Boquest, A.C., Shahdadfar, A., Fronsdal, K., Sigurjonsson, O., Tunheim, S. H., Collas, P., and Brinchmann, J. E. Isolation and transcription profiling of purified uncultured human stromal stem cells: Alteration of gene expression after in vitro cell culture. Molecular Biology of the Cell 16, 1131, 2005.

18. Planat-Benard, V., Silvestre, J. S., Cousin, B., Andre, M., Nibbelink, M., Tamarat, R., Clergue, M., Manneville, C., Saillan-Barreau, C., Duriez, M., Tedgui, A., Levy, B., Penicaud, L., and Casteilla, L. Plasticity of human adipose lineage cells toward endothelial cells - Physiological and therapeutic perspectives. Circulation 109, 656, 2004.

19. Miranville, A., Heeschen, C., Sengenes, C., Curat, C. A., Busse, R., and Bouloumie, A. Improvement of postnatal neovascularization by human adipose tissue-derived stem cells. Circulation 110, 349, 2004.

20. De Ugarte, D.A., Alfonso, Z., Zuk, P. A., Elbarbary, A., Zhu, M., Ashjian, P., Benhaim, P., Hedrick, M. H., and Fraser, J. K. Differential expression of stem cell mobilization-associated molecules on multi-lineage cells from adipose tissue and bone marrow. Immunology Letters 89, 267, 2003.

21. Gronthos, S., Franklin, D. M., Leddy, H. A., Robey, P. G., Storms, R. W., and Gimble, J. M. Surface protein characterization of human adipose tissue-derived stromal cells. Journal of Cellular Physiology 189, 54, 2001.

22. Katz, A.J., Tholpady, A., Tholpady, S. S., Shang, H. L., and Ogle, R. C. Cell surface and transcriptional characterization of human adipose-derived adherent stromal (hADAS) cells. Stem Cells 23, 412, 2005.

23. Dicker, A., Le Blanc, K., Astrom, G., van Harmelen, V., Gotherstrom, C., Blomqvist, L., Arner, P., and Ryden, M. Functional studies of mesenchymal stem cells derived from adult human adipose tissue. Experimental Cell Research 308, 283, 2005.

24. Brzoska, M., Geiger, H., Gauer, S., and Baer, P. Epithelial differentiation of human adipose tissue-derived adult stem cells. Biochemical and Biophysical Research Communications 330, 142, 2005.

25. Strem, B.M., Zhu, M., Alfonso, Z., Daniels, E. J., Schreiber, R., Begyui, R., MacLellan, W. R., Hedrick, M. H., and Fraser, J. K. Expression of cardiomyocytic markers on adipose tissue-derived cells in a murine model of acute myocardial injury. Cytotherapy 7, 282, 2005.

26. Lennon, D.P., Haynesworth, S. E., Young, R. G., Dennis, J. E., and Caplan, A. I. A Chemically-Defined Medium Supports In-Vitro Proliferation and Maintains the Osteochondral Potential of Rat Marrow-Derived Mesenchymal Stem-Cells. Experimental Cell Research 219, 211, 1995.

27. Boyan, B.D., Hummert, T. W., Dean, D. D., and Schwartz, Z. Role of material surfaces in regulating bone and cartilage cell response. Biomaterials 17, 137, 1996.

28. Olivieri, M.P., Rittle, K. H., Tweden, K. S., and Loomis, R. E. Comparative Biophysical Study of Adsorbed Calf Serum, Fetal Bovine Serum and Mussel Adhesive Protein Films. Biomaterials 13, 201, 1992.

29. Leong, D.T., Khor, W. M., Chew, F. T., Lim, T. C., and Hutmacher, D. W. Characterization of osteogenically induced adipose tissue-derived precursor cells in 2-dimensional and 3-dimensional environments. Cells Tissues Organs 182, 1, 2006.

## Claims

1. A method for enriching a stem cell-containing cell population and/or a progenitor cell-containing cell population for stem cells and/or progenitor cells, the method comprising:

   - providing said cell population with a scaffold comprising a copolymer comprising lactide and caprolactone,
   - allowing cells to attach to said scaffold, and
   - at least in part removing unattached cells.

2. A method according to claim 1, wherein said cell population are cells from adipose tissue.

3. A method according to claim 1 or 2, wherein said cell population comprises a stromal vascular fraction of adipose tissue.

4. A method according to claim 2 or 3, wherein said adipose cells have been obtained through resection and/or liposuction

5. A method according to any one of claims 1-4, wherein said scaffold comprises poly(D,L-lactide-co-caprolactone), poly(L,L-lactide-co-caprolactone) and/or a mixture thereof.

6. A method according to any one of claims 1-5, wherein the ratio lactide : caprolactone in the scaffold is in the range from about 50:50 to 90:10.

7. A method according to any one of claims 1-6, wherein said cells are allowed to attach to said scaffold during a time frame between ten minutes and one hour.

8. A method according to any one of claims 1-7, wherein said cells are allowed to attach to said scaffold during a time frame between ten minutes and thirty minutes.

9. A method according to any one of claims 1-8, further comprising inducing and/or enhancing proliferation of stem cells and/or progenitor cells attached to said scaffold.

10. A method according to any one of claims 1-9, comprising inducing and/or enhancing chondrogenic and/or osteogenic differentiation of stem cells and/or progenitor cells.

11. A scaffold comprising attached stem cells and/or progenitor cells obtainable by a method according to any one of claims 1-10.

12. A scaffold comprising:

- a porous matrix comprising a copolymer comprising at least one lactide and caprolactone, and
- stem cells and/or progenitor cells.

13. A scaffold according to claim 12, wherein said stem and/or progenitor cells cells are from adipose tissue.

14. A scaffold according to any one of claims 11-13, which is an artificial cartilage implant and/or bone implant.

15. An artificial cartilage implant and/or bone implant according to claim 14, further comprising at least one growth factor such as a TGF-β and/or a BMP, a polyamine and/or a steroid such as vitamin D and/or dexamethason.

16. An artificial cartilage implant and/or bone implant according to claim 14 or 15, wherein said scaffold comprises poly (D,L-lactide-co-caprolactone), poly(L,L-lactide-co-caprolactone) and/or a mixture thereof.

17. An artificial cartilage implant and/or bone implant according to any one of claims 14-16, wherein the ratio lactide : caprolactone in the scaffold is in the range from about 50:50 to 90:10.

18. An artificial cartilage implant and/or bone implant according to any one of claims 14-17, obtainable by a method according to any one of claims 1-10.

19. An artificial cartilage implant and/or bone implant according to any one of claims 14-18 for use as a medicament.

20. Use of an artificial cartilage implant and/or bone implant according to any one of claims 14-18 for the preparation of a medicament for treatment of cartilage damage and/or bone damage.

21. Use of an artificial cartilage implant and/or bone implant according to any one of claims 14-18 for cartilage-surgical operations and/or bone-surgical operations.

22. Use of an artificial cartilage implant and/or bone implant according to any one of claims 14-18 for inducing the growth of new cartilage tissue and/or bone tissue.

23. Use of a scaffold comprising a copolymer comprising lactide and caprolactone for enriching a stem cell-containing cell population and/or a progenitor cell-containing cell population for stem cells and/or progenitor cells.

24. A method for carrying out cartilage surgery and/or bone surgery, comprising implanting an artificial cartilage implant and/or bone implant according to any one of claims 14-18 into cartilage and/or a bone of a patient.

25. A method for inducing new cartilage tissue and/or bone tissue in a vertebrate in need thereof, the method comprising:

- removing adipose tissue from a vertebrate,
- providing cells from said adipose tissue to a scaffold comprising a copolymer comprising at least one lactide and caprolactone,
- allowing cells to attach to said scaffold,
- at least in part removing unattached cells, and
- introducing said scaffold into said vertebrate in need of new cartilage tissue and/or bone tissue.

Figure 1

Figure 2

Figure 3

Figure 4-I:

Figure 4-II:

Figure 5:

**European Patent Office**

## PARTIAL EUROPEAN SEARCH REPORT

**Application Number**

which under Rule 45 of the European Patent Convention shall be considered, for the purposes of subsequent proceedings, as the European search report

EP 06 07 6845

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AUNG TUN ET AL: "Chondroinduction of mouse mesenchymal stem cells in three-dimensional highly porous matrix scaffolds" JOURNAL OF BIOMEDICAL MATERIALS RESEARCH, vol. 61, no. 1, July 2002 (2002-07), pages 75-82, XP009086230 ISSN: 0021-9304 | 1,5,6, 9-25 | INV. C12N5/06 C12N5/08 A61L27/38 A61L27/56 C08L67/04 |
| Y | * page 76, left-hand column, paragraph 2 - page 77, left-hand column, paragraph 2 * <br> * page 78, left-hand column, paragraph 4 - page 81, right-hand column, paragraph 1 * <br> ----- <br> -/-- | 2-4,7,8 | |

| | TECHNICAL FIELDS SEARCHED (IPC) |
|---|---|
| | C12N A61L |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 9 July 2007 | Heiduschat, Carola |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................

& : member of the same patent family, corresponding document

EPO FORM 1503 03.82 (P04C07)

European Patent
Office

**PARTIAL EUROPEAN SEARCH REPORT**

Application Number

EP 06 07 6845

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | BURKS CHRIS A ET AL: "Characterization of 75:25 poly(l-lactide-co-epsilon-caprolactone) thin films for the endoluminal delivery of adipose-derived stem cells to abdominal aortic aneurysms." TISSUE ENGINEERING SEP 2006, vol. 12, no. 9, September 2006 (2006-09), pages 2591-2600, XP009086231 ISSN: 1076-3279 * page 2592, right-hand column, paragraph 1 - page 2593, left-hand column, paragraph 1 * * page 2595; figure 3 * * page 2595, left-hand column, paragraph 4 - page 2598, right-hand column, paragraph 1 * | 1-6, 11-13 | |
| | ----- | | |
| X | EP 1 454 641 A (ETHICON INC [US]) 8 September 2004 (2004-09-08) * paragraph [0015] * * paragraph [0042] * * paragraph [0048] * | 11-13 | **TECHNICAL FIELDS SEARCHED** (IPC) |
| | ----- | | |
| X | EP 1 493 404 A (DEPUY MITEK INC [US]) 5 January 2005 (2005-01-05) * paragraph [0013] * * paragraph [0072] * | 11-13 | |
| | ----- | | |
| X | EP 1 561 481 A (DEPUY MITEK INC [US]) 10 August 2005 (2005-08-10) | 11-13 | |
| Y | * paragraph [0036] - paragraph [0039] * * paragraph [0049] - paragraph [0051] * * paragraph [0056] * | 10,15 | |
| | ----- | | |
| | -/-- | | |

EPO FORM 1503 03.82 (P04C10)

**European Patent Office**

**PARTIAL EUROPEAN SEARCH REPORT**

Application Number

EP 06 07 6845

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| D,Y | ZUK P A ET AL: "Human adipose tissue is a source of multipotent stem cells" MOLECULAR BIOLOGY OF THE CELL, BETHESDA, MD, US, vol. 13, no. 12, 20 December 2002 (2002-12-20), pages 4279-4295, XP002249630 ISSN: 1059-1524 * page 4283, right-hand column, paragraph 2 * * page 4286, left-hand column, paragraph 3 - right-hand column, paragraph 2 * ----- | 2-4,10, 15 | |
| Y | JEONG S I ET AL: "In vivo biocompatibilty and degradation behavior of elastic poly(l-lactide-co-epsilon-caprolactone) scaffolds" BIOMATERIALS, ELSEVIER SCIENCE PUBLISHERS BV., BARKING, GB, vol. 25, no. 28, December 2004 (2004-12), pages 5939-5946, XP004512694 ISSN: 0142-9612 * the whole document * ----- | 1-25 | TECHNICAL FIELDS SEARCHED (IPC) |
| Y | LI W-J ET AL: "A three-dimensional nanofibrous scaffold for cartilage tissue engineering using human mesenchymal stem cells" BIOMATERIALS, ELSEVIER SCIENCE PUBLISHERS BV., BARKING, GB, vol. 26, no. 6, February 2005 (2005-02), pages 599-609, XP004523985 ISSN: 0142-9612 * page 601, left-hand column, paragraph 3 - right-hand column, paragraph 1 * * page 605, right-hand column, paragraph 1 - page 607, right-hand column, paragraph 1 * ----- | 1-25 | |

EPO FORM 1503 03.82 (P04C10)

**INCOMPLETE SEARCH
SHEET C**

Although claims 21, 22, 24 and 25 are directed to a method of treatment
of the human/animal body (Article 52(4) EPC), the search has been carried
out and based on the alleged effects of the compound/composition.

-----

**ANNEX TO THE EUROPEAN SEARCH REPORT
ON EUROPEAN PATENT APPLICATION NO.**                    EP 06 07 6845

This annex lists the patent family members relating to the patent documents cited in the above-mentioned European search report.
The members are as contained in the European Patent Office EDP file on
The European Patent Office is in no way liable for these particulars which are merely given for the purpose of information.

09-07-2007

| Patent document cited in search report | | Publication date | Patent family member(s) | | Publication date |
|---|---|---|---|---|---|
| EP 1454641 | A | 08-09-2004 | AU | 2004200531 A1 | 02-09-2004 |
| | | | CA | 2457460 A1 | 11-08-2004 |
| | | | JP | 2004243125 A | 02-09-2004 |
| | | | US | 2004156878 A1 | 12-08-2004 |
| | | | US | 2006263408 A1 | 23-11-2006 |
| EP 1493404 | A | 05-01-2005 | AU | 2004202223 A1 | 13-01-2005 |
| | | | CA | 2472702 A1 | 30-12-2004 |
| | | | JP | 2005021685 A | 27-01-2005 |
| | | | US | 2004267362 A1 | 30-12-2004 |
| EP 1561481 | A | 10-08-2005 | AU | 2005200305 A1 | 25-08-2005 |
| | | | CA | 2496184 A1 | 09-08-2005 |
| | | | JP | 2005237956 A | 08-09-2005 |
| | | | US | 2005177249 A1 | 11-08-2005 |

EPO FORM P0459

For more details about this annex : see Official Journal of the European Patent Office, No. 12/82

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Non-patent literature cited in the description

- **WUISMAN ; SMIT.** *Eur Spine J.,* 2006, vol. 15, 133-148
- **BONEWALD et al.** Principles of Bone Biology. Academic Press, 2002, 903-18
- **ROSEN ; WOZNEY et al.** Principles of Bone Biology. Academic Press, 2002, 919-28
- **REDDI.** *Tissue Eng.,* 2000, vol. 6, 351-9
- **BODEN.** *Tissue Eng.,* 2000, vol. 6, 383-99
- **DEUEL et al.** *Arch. Biochem. Biophys.,* 2002, vol. 397, 162-172
- **CAPLAN, A.I.** Mesenchymal Stem-Cells. *Journal of Orthopaedic Research,* 1991, vol. 9, 641
- **BERESFORD, J.N ; BENNETT, J. H ; DEVLIN, C ; LEBOY, P. S ; OWEN, M. E.** Evidence for An Inverse Relationship Between the Differentiation of Adipocytic and Osteogenic Cells in Rat Marrow Stromal Cell-Cultures. *Journal of Cell Science,* 1992, vol. 102, 341
- **BERRY, L ; GRANT, M. E ; MCCLURE, J ; ROONEY, P.** Bone-Marrow-Derived Chondrogenesis Invitro. *Journal of Cell Science,* 1992, vol. 101, 333
- **PITTENGER, M.F ; MACKAY, A. M ; BECK, S. C ; JAISWAL, R. K ; DOUGLAS, R ; MOSCA, J. D ; MOORMAN, M. A ; SIMONETTI, D. W ; CRAIG, S ; MARSHAK, D. R.** Multilineage potential of adult human mesenchymal stem cells. *Science,* 1999, vol. 284, 143
- **HALVORSEN, Y.D.C ; BOND, A ; SEN, A ; FRANKLIN, D. M ; LEA-CURRIE, Y. R ; SUJKOWSKI, D ; ELLIS, P. N ; WILKISON, W. O ; GIMBLE, J. M.** Thiazolidinediones and glucocorticoids synergistically induce differentiation of human adipose tissue stromal cells: Biochemical, cellular, and molecular analysis. *Metabolism-Clinical and Experimental,* 2001, vol. 50, 407
- **MIZUNO, H ; ZUK, P. A ; ZHU, M ; LORENZ, H. P ; BENHAIM, P ; HEDRICK, M. H.** Myogenic differentiation by human processed lipoaspirate cells. *Plastic and Reconstructive Surgery,* 2002, vol. 109, 199
- **ZUK, P.A ; ZHU, M ; MIZUNO, H ; HUANG, J ; FUTRELL, J. W ; KATZ, A. J ; BENHAIM, P ; LORENZ, H. P ; HEDRICK, M. H.** Multilineage cells from human adipose tissue: Implications for cell-based therapies. *Tissue Engineering,* 2001, vol. 7, 211
- **ZUK, P.A ; ZHU, M ; ASHJIAN, P ; DE UGARTE, D. A ; HUANG, J. I ; MIZUNO, H ; ALFONSO, Z. C ; FRASER, J. K ; BENHAIM, P ; HEDRICK, M. H.** Human adipose tissue is a source of multipotent stem cells. *Molecular Biology of the Cell,* 2002, vol. 13, 4279
- **WICKHAM, M.Q ; ERICKSON, G. R ; GIMBLE, J. M ; VAIL, T. P ; GUILAK, F.** Multipotent stromal cells derived from the infrapatellar fat pad of the knee. *Clinical Orthopaedics and Related Research,* 2003, vol. 196
- **ASHJIAN, P.H ; ELBARBARY, A. S ; EDMONDS, B ; DEUGARTE, D ; ZHU, M ; ZUK, P. A ; LORENZ, H. P ; BENHAIM, P ; HEDRICK, M. H.** In vitro differentiation of human processed lipoaspirate cells into early neural progenitors. *Plastic and Reconstructive Surgery,* 2003, vol. 111, 1922
- **SAFFORD, K.M ; HICOK, K. C ; SAFFORD, S. D ; HALVORSEN, Y. D. C ; WILKISON, W. O ; GIMBLE, J. M ; RICE, H. E.** Neurogenic differentiation of murine and human adipose-derived stromal cells. *Biochemical and Biophysical Research Communications,* 2002, vol. 294, 371
- **RANGAPPA, S ; FEN, C ; LEE, E. H ; BONGSO, A ; WEI, E. S. K.** Transformation of adult mesenchymal stem cells isolated from the fatty tissue into cardiomyocytes. *Annals of Thoracic Surgery,* 2003, vol. 75, 775
- **SEO, M.J ; SUH, S. Y ; BAE, Y. C ; JUNG, J. S.** Differentiation of human adipose stromal cells into hepatic lineage in vitro and in vivo. *Biochemical and Biophysical Research Communications,* 2005, vol. 328, 258
- **COWAN, C.M ; SHI, Y. Y ; AALAMI, O. O ; CHOU, Y. F ; MARI, C ; THOMAS, R ; QUARTO, N ; CONTAG, C. H ; WU, B ; LONGAKER, M. T.** Adipose-derived adult stromal cells heal critical-size mouse calvarial defects. *Nature Biotechnology,* 2004, vol. 22, 560
- **HELDER, M.N. ; KNIPPENBERG, M ; KLEIN-NULEND, J ; WUISMAN, P. I. J. M.** Stem cells from adipose tissue allow challenging new concepts for regenerative medicine. *Tissue Engineering accepted,* 2006
- **ISHAUG-RILEY, S.L ; OKUN, L. E ; PRADO, G ; APPLEGATE, M. A ; RATCLIFFE, A.** Human articular chondrocyte adhesion and proliferation on synthetic biodegradable polymer films. *Biomaterials,* 1999, vol. 20, 2245

- **BOQUEST, A.C ; SHAHDADFAR, A ; FRONSDAL, K ; SIGURJONSSON, O ; TUNHEIM, S. H ; COLLAS, P ; BRINCHMANN, J. E.** Isolation and transcription profiling of purified uncultured human stromal stem cells: Alteration of gene expression after in vitro cell culture. *Molecular Biology of the Cell,* 2005, vol. 16, 1131
- **PLANAT-BENARD, V ; SILVESTRE, J. S ; COUSIN, B ; ANDRE, M ; NIBBELINK, M ; TAMARAT, R ; CLERGUE, M ; MANNEVILLE, C ; SAILLAN-BARREAU, C ; DURIEZ, M.** Plasticity of human adipose lineage cells toward endothelial cells - Physiological and therapeutic perspectives. *Circulation,* 2004, vol. 109, 656
- **MIRANVILLE, A ; HEESCHEN, C ; SENGENES, C ; CURAT, C. A ; BUSSE, R ; BOULOUMIE, A.** Improvement of postnatal neovascularization by human adipose tissue-derived stem cells. *Circulation,* 2004, vol. 110, 349
- **DE UGARTE, D.A ; ALFONSO, Z ; ZUK, P. A ; EL-BARBARY, A ; ZHU, M ; ASHJIAN, P ; BENHAIM, P ; HEDRICK, M. H ; FRASER, J. K.** Differential expression of stem cell mobilization-associated molecules on multi-lineage cells from adipose tissue and bone marrow. *Immunology Letters,* 2003, vol. 89, 267
- **GRONTHOS, S ; FRANKLIN, D. M ; LEDDY, H. A ; ROBEY, P. G ; STORMS, R. W ; GIMBLE, J. M.** Surface protein characterization of human adipose tissue-derived stromal cells. *Journal of Cellular Physiology,* 2001, vol. 189, 54
- **KATZ, A.J ; THOLPADY, A ; THOLPADY, S. S ; SHANG, H. L ; OGLE, R. C.** Cell surface and transcriptional characterization of human adipose-derived adherent stromal (hADAS) cells. *Stem Cells,* 2005, vol. 23, 412
- **DICKER, A ; LE BLANC, K ; ASTROM, G ; VAN HARMELEN, V ; GOTHERSTROM, C ; BLOMQVIST, L ; ARNER, P ; RYDEN, M.** Functional studies of mesenchymal stem cells derived from adult human adipose tissue. *Experimental Cell Research,* 2005, vol. 308, 283
- **BRZOSKA, M ; GEIGER, H ; GAUER, S ; BAER, P.** Epithelial differentiation of human adipose tissue-derived adult stem cells. *Biochemical and Biophysical Research Communications,* 2005, vol. 330, 142
- **STREM, B.M ; ZHU, M ; ALFONSO, Z ; DANIELS, E. J ; SCHREIBER, R ; BEGYUI, R ; MACLELLAN, W. R ; HEDRICK, M. H ; FRASER, J. K.** Expression of cardiomyocytic markers on adipose tissue-derived cells in a murine model of acute myocardial injury. *Cytotherapy,* 2005, vol. 7, 282
- **LENNON, D.P ; HAYNESWORTH, S. E ; YOUNG, R. G ; DENNIS, J. E ; CAPLAN, A. I.** A Chemically-Defined Medium Supports In-Vitro Proliferation and Maintains the Osteochondral Potential of Rat Marrow-Derived Mesenchymal Stem-Cells. *Experimental Cell Research,* 1995, vol. 219, 211
- **BOYAN, B.D ; HUMMERT, T. W ; DEAN, D. D ; SCHWARTZ, Z.** Role of material surfaces in regulating bone and cartilage cell response. *Biomaterials,* 1996, vol. 17, 137
- **OLIVIERI, M.P ; RITTLE, K. H ; TWEDEN, K. S. ; LOOMIS, R. E.** Comparative Biophysical Study of Adsorbed Calf Serum, Fetal Bovine Serum and Mussel Adhesive Protein Films. *Biomaterials,* 1992, vol. 13, 201
- **LEONG, D.T ; KHOR, W. M ; CHEW, F. T ; LIM, T. C ; HUTMACHER, D. W.** Characterization of osteogenically induced adipose tissue-derived precursor cells in 2-dimensional and 3-dimensional environments. *Cells Tissues Organs,* 2006, vol. 182, 1